# NEUE EUROPÄISCHE PATENTSCHRIFT

(11) **EP 0 659 276 B2**
(45) Veröffentlichungstag und Bekanntmachung des Hinweises auf die Entscheidung über den Einspruch: **30.07.2008**
(45) Hinweis auf die Patenterteilung: 14.01.1998
(21) Anmeldenummer: 93917678.0
(22) Anmeldetag: 28.07.1993
(51) Int. Cl.: G01N 33/68, C07K 7/06, C07K 7/08, C12P 21/08, A61K 39/395

(54) **IMMUNOASSAY ZUM NACHWEIS VON KOLLAGEN ODER KOLLAGENFRAGMENTEN**
IMMUNOASSAY FOR DETECTING COLLAGEN OR COLLAGEN FRAGMENTS
DOSAGE IMMUNOLOGIQUE SERVANT A DETECTER LA PRESENCE DE COLLAGENE OU DE FRAGMENTS DE COLLAGENE

(30) Priorität: 29.07.1992 DE 4225038
(43) Veröffentlichungstag der Anmeldung: 28.06.1995
(73) Patentinhaber: Roche Diagnostics GmbH, 68305 Mannheim (DE)
(72) Erfinder: NASER, Werner, D-82362 Weilheim (DE); HUBER, Erasmus, D-86923 Finning (DE); SEIDEL, Christoph, D-82362 Weilheim (DE); ESSIG, Ulrich, D-82152 Planegg (DE)
(74) Vertreter: Dey, Michael
(86) Internationale Anmeldenummer: PCT/EP1993/002010
(87) Internationale Veröffentlichungsnummer: WO 1994/003813

(56) Entgegenhaltungen:
- EP-A- 0 298 210
- EP-A- 0 339 443
- EP-A- 0 465 104
- EP-A- 0 505 210
- WO-A-90/08195
- WO-A-91/09113
- WO-A-93/15107
- BIOCHEMISTRY Bd. 22 , 1983 Seiten 5213 - 5223 M.P.BERNARD ET AL. 'Nucleotide Sequences of Complementary Deoxyribonucleic Acids for the Pro alpha 1 Chain of Human Type I Procollagen. Statistical Evaluation of Structures That Are Conserved during Evolution.' in der Anmeldung erwähnt
- BIOCHEMISTRY Bd. 9, Nr. 24 , 1970 Seiten 4699 - 4706 E.M.CLICK ET AL. 'Isolation and Characterization of the Cyanogen Bromide Peptides from the alpha 1 and alpha 2 Chains of Human Skin Collagen,' in der Anmeldung erwähnt

### SEQUENZPROTOKOLL

(2) INFORMATION ZU SEQ ID NO: 1:
   (i) SEQUENZ CHARAKTERISTIKA:
      (A) LÄNGE: 9 Aminosäuren
      (B) ART: Aminosäure
      (D) TOPOLOGIE: linear
   (ii) ART DES MOLEKÜLS: Peptid
   (xi) SEQUENZBESCHREIBUNG: SEQ ID NO: 1:
   Xaa Phe Asp Phe Ser Phe Leu Pro Xaa
   1 5
(2) INFORMATION ZU SEQ ID NO: 2:
   (i) SEQUENZ CHARAKTERISTIKA:
      (A) LÄNGE: 16 Aminosäuren
      (B) ART: Aminosäure
      (D) TOPOLOGIE: linear
   (ii) ART DES MOLEKÜLS: Peptid
   (xi) SEQUENZBESCHREIBUNG: SEQ ID NO: 2:
   Ser Ala Gly Phe Asp Phe Ser Phe Leu Pro Gln Pro Pro Gln Glu Lys
   1 5 10 15
(2) INFORMATION ZU SEQ ID NO: 3:
   (i) SEQUENZ CHARAKTERISTIKA
      (A) LÄNGE: 13 Aminosäuren
      (B) ART: Aminosäure
      (D) TOPOLOGIE: linear
   (ii) ART DES MOLEKÜLS:.Peptid
   (xi) SEQUENZBESCHREIBUNG: SEQ ID NO: 3:
   Cys Gly Ser Ala Gly Phe Asp Phe Ser Phe Leu Pro Gln
   1 5 10

## Beschreibung

Die Erfindung betrifft einen kompetitiven Immunoassay zum Nachweis von Kollagen Typ I.

Kollagen stellt ein wichtiges Strukturprotein im Bindegewebe von Haut, Knorpel und Knochen dar. Bekannt sind 11 Typen, die jeweils aus drei Ketten bestehen. Jeder Typ setzt sich aus 1 - 3 verschiedenen Ketten zusammen, die als α1, α2 und α3 bezeichnet werden (E. Miller et al. in Methods in Enzymology 144, Structural and Contractile Proteins, ed. L. Cunningham, Academic Press Inc, 1987, p. 3 - 41). Eine charakteristische Eigenschaft des reifen Kollagens bestimmter Gewebe, wie insbesondere Knochen oder Knorpel, ist die Quervernetzung von nebeneinander liegenden Fasern durch Hydroxylysylpyridinolin oder Lysylpyridinolin (D. Fujimoto et al., J. Biochem 83 (1978), 863- 867; D. Eyre et al., Ann. Rev. Biochem 53 (1984), 717-748 und D. Eyre, Methods in Enzymology 144 (1987), 115-139). Diese Quervernetzungen können als biochemischer Marker für den spezifischen Nachweis von Kollagen genutzt werden (Z. Gunja-Smith et al., Biochem. J. 197 (1981), 759-762). Beim Abbau von extrazellulärem Kollagen gelangen Hydroxylysylpyridinolin- oder Lysylpyridinolin-Derivate, welche Peptidseitenketten enthalten oder freie Pyridinolin-Derivate mit Lysyl- oder Hydroxylysylresten, wie in der WO 91/10141 beschrieben, in Körperflüssigkeiten wie Blut oder Urin. Der Nachweis dieser Verbindungen in Körperflüssigkeiten ergibt daher Hinweise auf den Abbau von extrazellulärem Kollagen, wie er z.B. bei der Osteoporose sowie infolge von Tumoren des Knochengewebes auftritt. Zum Nachweis von solchen Hydroxylysyl- oder Lysylpyridinolinen mit Peptidseitenketten wurden in der WO 89/12824 monoklonale Antikörper beschrieben, welche durch Immunisierung mit entsprechenden quervernetzten Kollagenfragmenten erhalten wurden, die aus dem Urin isoliert werden können. Auch bei dem in WO 91/08478 beschriebenen Verfahren erfolgt der Nachweis von Kollagen über einen Antikörper gegen natürliche, d.h. in vivo erzeugte quervernetzte Abbauprodukte von Kollagen.

Ein Nachteil dieser aus natürlichen Quellen isolierten Peptide ist, daß keine sichere Quelle für eine reproduzierbare Herstellung der Antigene oder Bindepartner im Test vorhanden ist. Ein weiterer Nachteil der aus natürlichen Quellen isolierten Peptide ist die Gefahr einer Kontamination mit infektiösem Material.

Definierte Antigene können z.B. durch chemische Synthese eines Peptids erhalten werden, welches einem Epitop des Antigens entspricht. Werden hierfür kleine Peptide mit einem Molekulargewicht von etwa 700 - 1500 D verwendet, so ist die Bindung an ein Trägermolekül erforderlich, um ein Antigen mit immunogener Wirkung zu erhalten. Durch die Bindung an das Trägermolekül darf dabei die Struktur des Epitops nicht verändert werden. Die Kupplung an das Trägermolekül erfolgt daher bislang bevorzugt an den Enden der Peptidkette in ausreichendem Abstand von dem vermuteten Epitopbereich (Laboratory Technics in Biochemistry and Molecular Biology, Synthetic Polypeptides as Antigens, Editors R.H. Burdon und P.H. van Knippenberg, Elsevier, Amsterdam, New York, Oxford 1988, Seiten 95-100).

Ein Problem bei der chemischen Synthese eines definierten Antigens, das einem natürlichen Abbauprodukt von quervernetztem Kollagen entspricht, stellt die aus der Quervernetzung resultierende Hydroxylysyl- oder LysylpyridinolinStruktur dar, deren chemische Synthese bislang nicht gelungen ist.

Aufgabe der Erfindung war es daher, ein definiertes Antigen zur Herstellung von Antikörpern gegen Kollagen Typ I oder Kollagenfragment davon, zum Einsatz als spezifischer Bindepartner des Antikörpers gegen Kollagen oder Kollagenfragmente in einem kompetitiven Immunoassay und als Standardmaterial zur Erstellung einer Standard- oder Eichkurve in einem kompetitiven Immunoassay zum Nachweis von Kollagen oder Kollagenfragmenten vom Typ I zur Verfügung zu stellen.

Bisher wurde immer davon ausgegangen, daß es zum Nachweis der Kollagen- oder Kollagenabbauprodukte in einer Probe notwendig sei, die Quervernetzungsstrukturen an sich oder sogenannte quervernetzte Peptide, die durch die Quervernetzung der Hydroxylysyl- oder Lysylreste zustande kommen, nachzuweisen, da diese Hydroxylysyl- oder Lysylpyridinolinstruktur charakteristisch für Kollagen ist. Beispiele für solche Nachweismethoden sind in der WO 89/12824, WO 91/08478, WO 89/04491 und der WO 91/10141 beschrieben.

Es wurde nun überraschenderweise gefunden, daß es zur Lösung der oben genannten Aufgabe ausreicht, ein definiertes Antigen, einen Bindepartner oder ein Standardmaterial zu verwenden, das ein synthetisches lineares Pepid enthält, das einer Sequenz des nicht-helikalen linearen N-terminalen Bereiches von Kollagen Typ I oder der Sequenz SEQ ID NO 1 aus dem nicht-helikalen C-terminalen Bereich von Kollagen Typ I entspricht und keine Hydroxylysyl- oder Lysylpyridinolin-Quervernetzung enthält. Die Vorteile bei Verwendung von synthetischen linearen Peptiden zur Verwendung als Bindepartner in Immunoassays, als Standardmaterial oder als Immunogen bei der Antikörper-Herstellung liegen darin, daß diese Peptide, im Gegensatz zu Peptiden aus natürlichen Quellen, in genau definierter Struktur reproduzierbar hergestellt werden können. Zudem zeigt ein Immunoassay, in dem solche kurzen synthetischen Peptide eingesetzt werden, eine geringere Störanfälligkeit.

Gegenstand der Erfindung ist daher ein kompetitiver Immunoassay zum Nachweis von Kollagen Typ I oder Kollagenfragmenten davon in einer Probe, der dadurch gekennzeichnet ist, daß ein Bindepartner, der ein synthetisches lineares Peptid enthält, das einer Sequenz des nicht-helikalen linearen N-terminalen Bereiches von Kollagen Typ I oder der Sequenz SEQ ID NO 1 aus dem nicht-helikalen C-terminalen Bereich von Kollagen Typ I entspricht und keine Hydroxylysyl- oder Lysylpyridinolin-Quervernetzung enthält, mit einem Antikörper, der das synthetische lineare Peptid zu binden vermag, und der Probe inkubiert und die Bindung des Antikörpers an den Bindepartner in geeigneter Weise bestimmt wird.

Ein weiterer Gegenstand der Erfindung ist die Verwendung eines Antigens, das ein synthetisches lineares Peptid enthält, das einer Sequenz des nicht-helikalen N-terminalen Bereichs von Kollagen Typ I oder der Sequenz SEQ ID NO 1 aus dem nicht-helikalen C-terminalen Bereich von Kollagen Typ I entspricht und keine Hydroxylysyl- oder Lysylpyridinolin-Quervernetzung enthält, als Standardmaterial zur Erstellung einer Standard- oder Eichkurve in einem kompetitiven Immunoassay zum Nachweis von Kollagen Typ I oder Kollagenfragmenten davon.

Als synthetische lineare Peptide sind alle zusammenhängenden Aminosäuresequenzen des nicht-helikalen N-terminalen Bereichs von Kollagen Typ I oder die Sequenz SEQ ID NO 1 aus dem nicht-helikalen C-terminalen Bereich von Kollagen Typ I, die keine Hydroxylysyl- oder Lysylpyridinolin-Quervernetzung enthalten, geeignet. Diese Bereiche sind aus Chu et al., Nature 310, 337-340 (1984), Click et al., Biochemistry 9, 4699-4706 (1970), Morgan et al., J. Biol. Chem. 245, 5042-5048 (1970) und Bernard et al., Biochemistry 22, 5213-5223 (1983) bekannt. Bevorzugt werden Peptide aus mindestens 5 Aminosäuren, besonders bevorzugt aus mindestens 8 Aminosäuren, verwendet. Dabei ist es nicht notwendig, daß die Sequenz den Bereich der Quervernetzung umfaßt. Sie kann aber sehr wohl ebenfalls mit diesem Bereich überlappen. In keinem Fall liegt aber in dem synthetischen Peptid eine Hydroxylysyl- oder Lysylpyridinolin-Quervernetzung vor. Das Peptid mit der in SEQ ID NO 1 gezeigten Sequenz aus dem C-terminalen Bereich der aα1-Kette von Kollagen ist besonders geeignet.

Die Konzentration von Abbauprodukten von Kollagen ist ein wichtiger diagnostischer Marker für das Ausmaß einer Osteolyse. Mit Hilfe der synthetischen linearen Peptide ist es möglich, einen kompetitiven Immunoassay zum Nachweis von Kollagen oder Kollagenfragmenten durchzuführen. Es hat sich überraschenderweise gezeigt, daß diese Peptide sehr gut mit den Kollagenfragmenten, die in natürlichen Proben wie Plasma, Serum oder Urin vorkommen, um Antikörper gegen diese Kollagenfragmente konkurrieren und damit einen kompetitiven Test ermöglichen. Solche Antikörper gegen Kollagen oder Kollagenabbauprodukte sind kommerziell erhältlich, beispielsweise in dem Telopeptide ICTP [¹²⁵J) Radioimmunoassay Kit der Firma Orion Diagnostica, Finnland. Sie können aber ebenfalls erfindungsgemäß mittels des synthetischen linearen Peptids hergestellt werden. WO-A-90/08195 beschreibt einen Test zum Nachweis von Typ IX Kollagen, durch einen monoklonalen Antikörper, der mittels synthetischer Peptide, die Sequenzen der globulären Domäne NC 4 darstellen, hergestellt wurde. Die Verhältnisse bei Kollagen IX ist nicht mit Kollagen Typ 1 zu verglichen; insbesondere tritt in der globulären Domäne bei Typ IX Kollagen keine charakteristische Quervernetzung auf. In EP-A-0298210 wird zum Immunisieren von Tieren eine Peptidsequenz verwendet, um Antikörper zu gewinnen, die Prokollagen vom Typ III erkennen. Prokollagen stellt keinen diagnostischen Marker für die Osteolyse dar.

Zum Einsatz in einem kompetitiven Immunoassay kann das synthetische lineare Peptid direkt als Bindepartner, der an eine Festphase gebunden ist, benutzt werden oder aber gekoppelt an eine zweite Komponente. Die Kopplung an die zweite Komponente erfolgt bevorzugt über die N- und C-terminale Aminosäuren des linearen Peptids. Gegebenenfalls kann zwischen dem Peptid und der zweiten Komponente noch ein Spacer eingefügt werden. Die zweite Komponente kann beispielsweise dazu dienen, das Peptid indirekt an eine Festphase zu koppeln. Beispiele hierfür sind dem Fachmann bekannt. Bevorzugt wird das Peptid an Rinderserumalbumin gekoppelt und das Kopplungsprodukt adsorptiv an eine Festphase zum Beispiel ein Kunststoffröhrchen gebunden. Das Peptid kann auch an Biotin kovalent gebunden werden. Die Anheftung an die Festphase erfolgt dann über die Bindung an Avidin oder Streptavidin, das seinerseits an die Festphase gebunden wurde. Die zweite Komponente kann auch als Träger für mehrere Peptide dienen, zum Beispiel in einem kompetitiven turbidimetrischen Inhibierung-Immunoassay (TINIA), wobei mehrere Peptide an beispielsweise Albumin, Immunglobuline, β-Galaktosidase, Polymere wie Polylysin oder Dextranmoleküle wie in der EP-A-0 545 350 beschrieben oder Partikel wie Latex gekoppelt sind. Vorzugsweise werden 30 bis 40 Peptidmoleküle je Trägermolekül gekoppelt. Das Peptid kann auch an eine Komponente, die eine Markierung darstellt, gekoppelt sein. Beispiele für all diese Testvarianten sind dem Fachmann bekannt.

Bei der Testführung kann der Antikörper gleichzeitig oder sequenziell mit der Probe sowie dem Bindepartner, der das synthetische lineare Peptid enthält, inkubiert werden. Anschließend wird in üblicher Weise die Menge des gebundenen oder nichtgebundenen Antikörpers bestimmt. Als kompetitive Testvarianten zur Bestimmung der Menge an gebundenem oder ungebundenem Antikörper können beispielsweise Agglutinationstests, wie TINIA, oder FPIA- (Fluoreszenz-Polarisations-Immunoassay) (W. Dandliker et al., J.Exp.Med. 122 (1965), 1029), EMIT- (Enzyme Multiplied Immunoassay) (Gunzer et al., Kontakte III (1980), 3-11) und CEDIA-Prinzipien (Henderson et al., Clinical Chemistry 32 (1986), 1637-1641) dienen. Die erfindungsgemäßen Peptide haben sich als besonders geeignet für die Verwendung als definierte Bindepartner zur Kompetition mit der Probe um die Bindung an die Antikörper erwiesen. Besonders bevorzugt ist das synthetische lineare Peptid mit der in SEQ ID NO 1 gezeigten Sequenz.

Nach der Bestimmung des Ausmaßes der Bindung des Antikörpers an den Bindepartner, das ein Maß für die Menge an Antigen in der Probe darstellt, kann die genaue Menge an Antigen in der Probe in üblicher Weise durch Vergleich mit in gleicher Weise behandeltem Standard ermittelt werden.

Als Standard können Kollagenabbauprodukte isoliert aus natürlichem Material verwendet werden. Diese zeichnen sich jedoch naturgemäß durch eine bestimmte Schwankung aus. Geeigneter erwiesen hat sich als Standardmaterial ein Antigen, das das erfindungsgemäße synthetische lineare Peptid enthält. Das Antigen des Standards kann dabei lediglich aus diesem Peptid bestehen oder aber aus diesem Peptid, das an einen geeigneten Träger gekoppelt ist, der beispielsweise zur besseren Wasserlöslichkeit des Peptids dient. Zur Herstellung des Standardmaterials aus Peptid und Träger wird das lineare Peptid, das einer Sequenz des nicht-helikalen N-terminalen Bereichs von Kollagen Typ I oder der Sequenz SEQ ID NO 1 aus dem nicht-helikalen C-terminalen Bereich von Kollagen Typ I entspricht und keine Hydroxylysyl- oder Lysylpyridinolin-Quervernetzung enthält, synthetisiert und über seine N- oder C-terminale Aminosäure durch geeignete Kopplungsmethoden an das Trägermolekül gebunden. Es können ein oder mehrere Peptide pro Trägermolekül gebunden werden. Gegebenenfalls kann die Kopplung über einen Spacer erfolgen. Für bestimmte Zwecke, wie beispielsweise für Agglutinationstests, kann es von Vorteil sein, mehrere erfindungsgemäße Peptide unterschiedlicher Sequenz an ein Trägermolekül zu binden, vor allem falls polyklonale Antikörper im Test eingesetzt werden, die nicht mit Hilfe des erfindungsgemäßen Antigens hergestellt wurden und damit meist mehrere Epitope erkennen.

Als Antikörper in dem kompetitiven Immunoassay können die bereits bekannten Antikörper gegen Kollagenabbauprodukte verwendet werden. Geeignet sind auch Antikörper, die mit Hilfe eines Antigens, das das erfindungsgemäße lineare synthetische Peptid enthält, gewonnen wurden.

Zur Immunisierung müssen die linearen synthetischen Peptide, die einer oder mehrerer Sequenzen des nicht-helikalen C- oder N-terminalen Bereichs von Kollagen Typ I entprechen, an ein geeignetes Trägerprotein, wie zum Beispiel Keyhole Limpet Hemocyanine, Rinderserumalbumin oder Edestin, gebunden werden. Zur Herstellung dieser Antigene oder Immunogene werden zunächst die linearen Peptide in üblicher Weise chemisch synthetisiert. Anschließend erfolgt die Kopplung der synthetischen Peptide über die N-terminale Aminogruppe mittels Maleinimidohexansäure-N-hydroxysuccinimidester an die oben genannten Trägerproteine. Es hat sich gezeigt, daß zur Herstellung von Antikörpern, die für die kompetitive Testführung geeignet sind, synthetische lineare Peptide mit der in SEQ ID NO 1, 2 oder 3 gezeigten Sequenz besonders geeignet sind.

Mit den Antigenen, die ein synthetisches lineares Peptid enthalten, das einer Sequenz des nicht-helikalen C- oder N-terminalen Bereichs von Kollagen Typ I entspricht, ist es möglich, Antikörper zu erhalten, welche nicht nur das erfindungsgemäße Peptid, sondern auch die in Körperflüssigkeiten vorkommenden Abbauprodukte von Kollagen erkennen.

Die Herstellung von Antikörpern gegen Kollagen oder Kollagenfragmente kann durch Immunisierung mit einem erfindungsgemäßen Antigen und Isolierung des gewünschten Antikörpers aus dem Serum der immunisierten Tiere nach bekannten Verfahren erfolgen. Vorzugsweise erfolgt die Isolierung des gewünschten Antikörpers über Immunadsorption an einem an ein Trägerprotein, vorzugsweise Sepharose, gekoppelten Peptid der in SEQ ID NO 1, 2 oder 3 gezeigten Sequenz.

Die Herstellung monoklonaler Antikörper gegen Kollagen oder Kollagenfragmente kann durch Immunisierung mit einem erfindungsgemäßen Antigen, Immortalisierung der Milzzellen der immunisierten Tiere, Klonierung derjenigen immortalisierten Milzzellen, welche den gewünschten Antikörper produzieren und Isolierung des Antikörpers aus den klonierten Zellen oder dem Kulturüberstand dieser Zellen erfolgen.

Die Immunisierung erfolgt in den hierfür üblicherweise verwendeten Tieren, vorzugsweise werden Mäuse oder Kaninchen verwendet.

Die Immortalisierung der Milzzellen der immunisierten Tiere erfolgt nach dem Fachmann geläufigen Verfahren, wie z.B. der Hybridomtechnik (Köhler und Milstein, Nature 256 (1975), 495- 497) oder durch Transformation mit dem Epstein-Barr-Virus (EBV-Transformation). Zum Nachweis derjenigen immortalisierten Zellen, die den gewünschten Antikörper produzieren, wird eine Probe des Kulturüberstands in einem üblichen Immunoassay mit dem zur Immunisierung verwendeten erfindungsgemäßen Antigen inkubiert und untersucht, ob ein Antikörper an dieses Antigen bindet.

Polyklonale und monoklonale Antikörper reagieren nicht nur mit dem zur Immunisierung verwendeten erfindungsgemäßen Hapten, sondern gut mit Kollagen, sowie mit den in Körperflüssigkeiten gefundenen natürlichen Abbauprodukten von Kollagen.

Die Antikörper können daher in den oben beschriebenen Nachweisverfahren zur Bestimmung von Kollagen oder Kollagenfragmenten verwendet werden.

Die polyklonalen oder monoklonalen Antikörper können zur Bestimmung der Osteolyse durch Inkubation des Antikörpers mit einer Gewebeprobe und Bestimmung des an den Antikörper bindenden Kollagenabbauproduktes verwendet werden.

Die Erfindung wird durch die folgenden Beispiele in Verbindung mit den Sequenzprotokollen näher erläutert.

SEQ ID NO 1 zeigt die Sequenz eines erfindungsgemäßen Peptids aus 9 Aminosäuren, wobei Xaa eine beliebige Aminosäure bedeutet.

SEQ ID NO 2 zeigt die Sequenz eines Vergleichspeptids aus 16 Aminosäuren.

SEQ ID NO 3 zeigt die Sequenz eines Vergleichspeptids aus 10 Aminosäuren.

### Beispiel 1

### Peptidsynthesen (Vergleichsbeispiel)

Die eine Teilsequenz der Aminosäurensequenz von Kollagen aufweisenden Peptide der in den Sequenzprotokollen SEQ ID NO 2 und 3 gezeigten Sequenzen werden mittels Fluorenylmethyloxycarbonyl(Fmoc)-Festphasenpeptidsynthese an einem a) Labortec SP 640 Peptide Synthesizer bzw. b) Zinsser Analytic SMPS 350 Peptidsynthesizer hergestellt.

### a) Herstellung von Acetyl-Ser-Ala-Gly-Phe-Asp-Phe-Ser-Phe-Leu-Pro-Gln-Pro-Pro-Gln-Glu-Lys-Amid (SEQ ID NO 2)

In der angegebenen Reihenfolge werden jeweils 4,0 Äquivalente von folgenden Fmoc-Aminosäurederivaten eingesetzt:

| | |
|---|---|
| Lys | mit tert. Butyloxycarbonyl-Schutzgruppe |
| Glu | mit tert. Butylester-Schutzgruppe |
| Gln | ohne Seitenketten-Schutzgruppe |
| Pro | ohne Seitenketten-Schutzgruppe |
| Pro | ohne Seitenketten-Schutzgruppe |
| Gln | ohne Seitenketten-Schutzgruppe |
| Pro | ohne Seitenketten-Schutzgruppe |
| Leu | ohne Seitenketten-Schutzgruppe |
| Phe | ohne Seitenketten-Schutzgruppe |
| Ser | mit tert. Butylether-Schutzgruppe |
| Phe | ohne Seitenketten-Schutzgruppe |
| Asp | mit tert. Butylester-Schutzgruppe |
| Phe | ohne Seitenketten-Schutzgruppe |
| Gly | ohne Seitenketten-Schutzgruppe |
| Ala | ohne Seitenketten-Schutzgruppe |
| Ser | mit tert. Butylether-Schutzgruppe |
| Acetyl | Essigsäureanhydrid |

Die Aminosäuren oder Aminosäurederivate werden in N-Methylpyrrolidon gelöst.

Das Peptid wird an 3 g 4-(2',4'-Dimethoxyphenyl-Fmocaminomethyl)-phenoxy-Harz (Tetrahedron Letters 28 (1987), 2107) mit einer Beladung von 0,87 mmol/g synthetisiert (JACS 95 (1973), 1328). Die Kupplungsreaktionen werden bezüglich Fmoc-Aminosäurederivat mit 4,4 Äquivalenten Dicyclohexylcarbodiimid und 4,8 Äquivalenten N-Hydroxybenzotriazol in Dimethylformamid als Reaktionsmedium während 60 Minuten durchgeführt. Der Kupplungserfolg wird mittels Kaiser-Test (Anal. Biochem. 34 (1970), 595) an dem mit Isopropanol gewaschenen Syntheseharz kontrolliert. Sofern sich hierbei ein noch nicht vollständiger Umsatz ergibt, wird durch Nachkuppeln gemäß den oben angegebenen Bedingungen der Umsatz vervollständigt. Nach jedem Syntheseschritt wird die Fmoc-Gruppe mittels 20 %-igem Piperidin in Dimethylformamid in 20 Minuten abgespalten. Die Harzbeladung wird mittels UV-Adsorption der freigesetzten Fulven-Gruppe nach jeder Piperidinbehandlung ermittelt. Nach der Synthese beträgt die Beladung noch 0,68 mmol/g.

Die Freisetzung des Peptids vom Syntheseharz und die Abspaltung der säurelabilen Schutzgruppen erfolgt mit 80 ml Trifluoressigsäure, 5 ml Ethandithiol, 2,5 g Phenol, 2,5 ml m-Kresol und 5 ml Wasser in 60 Minuten bei Raumtemperatur.

Die Reaktionslösung wird anschließend im Vakuum eingeengt. Der Rückstand wird in Diisopropylether aufgenommen, 1/2 - 2 Stunden kräftig gerührt und dann abfiltriert. Das Material wird dann mittels einer Gelpermeationschromatographie an Sephadex G15 vorgereinigt, wobei als Elutionsmittel 0,5 %-ige Essigsäure verwendet wird. Das erhaltene Rohmaterial wird anschließend abfiltriert und mittels einer präparativen HPLC an Nucleosil RP18 (Säule 40 mm x 250 mm 300 Å, 5 µm) über einen Gradienten von 100 % Puffer A (Wasser, 0,1 % Trifluoressigsäure) zu 100 % Puffer B (60 % Acetonitril, 40 % Wasser, 0,1 % Trifluoressigsäure) in 120 Minuten isoliert. Die Identität des eluierten Materials wird mittels Fast-Atom-Bombardment-Massenspektrometrie (FAB-MS) geprüft.

### b) Herstellung von Ala-Gly-Phe-Asp-Phe-Ser-Phe-Leu-Pro-Gln (SEQ ID NO 3)

Das Peptid wurde an 30 mg 4-(2',4'-Dimethoxyphenyl-Fmoc-aminomethyl) phenoxy-Harz SA 5030 der Firma Advanced Chemtech mit einer Beladung von 0,47 mmol/g hergestellt. Von folgenden Fmoc-Aminosäure-Derivaten wurden zweimal je 140 µMol zusammen mit je 140 µMol 1-Hydroxybenzotriazol in Dimethylformamid DMF und 10 µMol N,N-Diisopropylcarbodiimid in DMF an das aufzubauende festphasengebundene Peptid angekoppelt:
Glu mit Trityl-Schutzgruppe
Ser mit tert. Butyl-Schutzgruppe
Asp mit tert. Butyl-Schutzgruppe

| | | |
|---|---|---|
| Pro | \| | |
| Leu | \| | |
| Phe | | > jeweils ohne Seitenketten-Schutzgruppe |
| Gly | \| | |
| Ala | \| | |

Die Kupplungszeiten betrugen 30 und 40 Minuten. Die Abspaltzeit betrug 20 Minuten und wurde mit einer Lösung von 50 % Piperidin in DMF durchgeführt. Die Waschschritte wurden mit DMF achtmal nach jedem Reaktionsschritt durchgeführt. Die Freisetzung des Peptids erfolgte durch Behandlung des vom Lösungsmittel abfiltrierten und mit Dichlormethan und Methanol gewaschenen Harz mit 1 ml einer Lösung von 90 % Trifluoressigsäure, 3 % Thioanisol, 3 % Ethandithiol und 3 % Thiokresol innerhalb von 20 Minuten und 140 Minuten. Das Produkt wurde durch Zugabe von 15 ml kaltem Diisopropylether zum vereinigten Filtrat ausgefällt und durch Filtration isoliert. Der Rückstand wurde in 50 %iger Essigsäure gelöst und lyophilisiert. Es wurden 8 mg weißes Lyophilisat einer Reinheit von 79 % laut HPLC erhalten. Die Identität wurde mittels FAB-Massenspektroskopie bestätigt.

### Beispiel 2

### Aktivierung von Peptiden (Vergleichsbeispiel)

Das gemäß Beispiel 1a) hergestellte Peptid wird durch Acylierung mit Maleinimidohexanoyl-N-hydroxysuccinimid (MHS) aktiviert. Dazu wird 0,1 mmol des Peptids in 20 ml 0,1 mol/l Kaliumphosphatpuffer pH 7,5 gelöst, mit einer Lösung von 0,1 mmol MHS in 6 ml Dioxan versetzt und 20 min. bei 20 °C gerührt. Anschließend wird der pH-Wert mit Eisessig auf pH 4 eingestellt und das Reaktionsgemisch sofort lyophilisiert. Das Lyophilisat wird in 5 ml Wasser gelöst und über präparative HPLC an einer Waters Delta-Pak® C18-Säule (100 Å, 15 µm 50 x 300 mm) über einen Elutionsgradienten von 100 % A (Wasser 0,1 % Trifluoressigsäure) zu 100 % B (99,9 % Acetonitril 0,1 % Trifluoressigsäure) aufgereinigt.

### Beispiel 3

### Herstellung von Immunogenen durch Kopplung von aktivierten Peptiden an Trägerproteine (Vergleichsbeispiel)

Beschrieben wird die Kopplung von aktivierten Peptiden an Keyhole Limpet Hemocyanine (KLH), Rinderserumalbumin (RSA) und β-Galaktosidase (βGal). Zur Kopplung der nach Beispiel 2 mit MHS aktivierten Peptide ist es erforderlich, daß das Trägerprotein freie SH-Gruppen trägt. βGal weist diese natürlicherweise bereits auf, erfordert also keine weitere Vorbehandlung. Bei KLH und RSA werden die NH₂-Gruppen der ε-Aminoseitenkette von Lysinresten durch Behandlung mit N-Succinimidyl-S-acetylthiopropionat (SATP) derivatisiert und dadurch in SH-Gruppen umgewandelt.

Man erhält somit ein Trägerprotein, das eine gegenüber dem nativen Zustand erhöhte Anzahl von SH-Gruppen aufweist. Hierfür wird zu einer Lösung aus 1,39 g KLH in 500 ml 0,1 mol/l Kaliumphosphatpuffer pH 8,5 innerhalb von 20 min. 113,51 mg SATP (in 10 ml Dioxan gelöst) zugetropft. Nach 30 min. Rühren bei 20 °C wird der pH-Wert der Reaktionslösung mit 0,1 mol/l Natronlauge auf pH 8,5 nachgestellt und weitere 24 Stunden gerührt. Die Lösung wird anschließend mit Hilfe einer Amiconzelle (Membran YM10) auf 100 ml aufkonzentriert, 3 x 24 Stunden gegen je 3 l 0,1 mol/l Kaliumphosphatpuffer pH 8,5/ 0,05 mol/l Natriumchlorid dialysiert und anschließend lyophilisiert.

Zur Abspaltung der S-Acetylschutzgruppe werden 481 mg des KLH-SATP Lyophilisats in 20 ml 0,1 mol/l Kaliumphosphatpuffer pH 8,5/0,05 mol/l Natriumchlorid gelöst, mit 0,5 ml frisch zubereiteter 1 mol/l Hydroxylaminlösung versetzt und 90 min. bei 20 °C gerührt.

7,23 mol des aus Beispiel 2 erhaltenen aktivierten Peptids in 4 ml Wasser werden zu dem derivatisierten Trägerprotein gegeben und 20 Stunden bei 20 ° C gerührt. Anschließend wird die trübe Lösung 2 x gegen 1 l 0,1 mol/l Kaliumphosphatpuffer pH 8,5/0,05 mol/l Natriumchlorid dialysiert. Das Dialysat wird zentrifugiert, der klare Überstand abdekantiert und lyophilisiert.

### Beispiel 4

### Herstellung polyklonaler Antikörper gegen lineare Kollagenfragmente (Vergleichsbeispiel)

Je 5 Schafe wurden in an sich bekannter Weise mit dem Immunogen aus Beispiel 3 immunisiert. Die Immunogene enthielten das Peptid mit der in SEQ ID NO 2 genannten Sequenz, das den Aminosäuren Nr. 892 bis 907 in der Sequenz der α-Kette von Kollagen Typ I entspricht. Als Trägerprotein diente KLH bzw. β-Galaktosidase. Die Tiere wurden in monatlichen Abständen mit den Immunogenen in kompletten Freundschen Adjuvans immunisiert. Die Dosis betrug 500 µg je Tier und Immunisierung. Vier Monate nach Erstimmunisierung wurden Blutproben entnommen und die erhaltenen Antikörper auf Reaktion mit Kollagenfragmenten geprüft.

**ELISA zum Nachweis der Reaktion der Antiseren mit Kollagenfragmenten**

Folgendes Material und Reagenzien wurden verwendet:

| Mikrotiterplatten Maxisorp F96, Firma Nunc | |
|---|---|
| Beschichtungspuffer | 50 mM Natriumcarbonat pH 9,6 |
| | 0,1 % NaN₃ |
| Inkubationspuffer | 10 mM Natriumphosphat pH 7,4 |
| | 0,1 % Tween 20 |
| | 0,9 % NaCl |
| | 1 % Rinderserumalbumin |
| Substratlösung | ABTS®, Boehringer Mannheim GmbH, Katalog Nr. |
| | 857424 |
| | Zur Verstärkung des Signals wurde der Lösung 2 |
| | mg/ml Vanillin zugesetzt. |
| Waschlösung | 0,1 % Tween 20 |
| | 0,9 % NaCl |
| | |

Die Vertiefungen der Titerplatten wurden mit je 100 µl einer Lösung gefüllt, die 10 µg/ml Kollagenfragmente in Beschichtungspuffer enthielt. Die Kollagenfragmente wurden entsprechend den Angaben in EP-A-0 505 210 durch Protease-Verdauung von humanem Kollagen aus Knochen hergestellt.

Nach einer Stunde Inkubation bei Raumtemperatur unter Schütteln wurde dreimal mit Waschlösung gewaschen.

Die Antiseren wurden mit Inkubationspuffer 1 : 4000 verdünnt und je 100 µl in den Vertiefungen der Mikrotiterplatte eine Stunde unter Schütteln bei Raumtemperatur inkubiert. Anschließend wurden die Vertiefungen dreimal mit Waschlösung gewaschen.

Ein Konjugat aus Meerrettich-Peroxidase mit Kaninchen-Antikörpem gegen den Fc-Teil von Schaf-IgG wird im Inkubationspuffer bis zur Konzentration von 12,5 mU/ml verdünnt und die Näpfe der Mikrotiterplatte mit je 100 ml davon beschickt. Nach einer Stunde Inkubation unter Schütteln bei Raumtemperatur werden die Titerplatten dreimal mit Waschlösung gewaschen.

100 µl Substratlösung werden hinzugefügt und inkubiert, bis eine Farbentwicklung deutlich wird (10 - 60 Minuten). Die Extinktion wird als Differenzmessung bei 405 und 492 nm erfaßt.

Die Seren der meisten Tiere zeigten eine starke Reaktion mit den Kollagenfragmenten auf der Festphase. Das Serum eines nicht immunisierten Tieres zeigte unter gleichen Bedingungen nur ein schwaches Meßsignal. Die Ergebnisse sind in der Tabelle 1 dargestellt.

**Tabelle 1**

| β-Galaktosidase | | KLH | |
|---|---|---|---|
| Tier Nr. | Extinktion | Tier Nr. | Extinktion |
| 1 | 1,05 | 1 | 1,16 |
| 2 | 1,18 | 2 | 2,62 |
| 3 | 1,49 | 3 | 1,28 |
| 4 | 0,48 | 4 | 1,42 |
| 5 | > 2,70 | 5 | 1,81 |

### Beispiel 5

### Bestimmung von Kollagen sowie dessen Abbauprodukten in Körperflüssigkeiten über einen kompetitiven Test (Vergleichsbeispiel)

Die Vertiefungen einer 96-er Mikrotiterplatte werden gemäß EP-A 0 344 578 mit Streptavidin (100 µl einer Lösung von 1 µg/ml in PBS) bei 4 °C über Nacht beschichtet und noch freie unspezifische Bindungsstellen durch Inkubation mit 300 µl BSA (Rinderserumalbumin, 10 mg/ml) für 2 Stunden bei Raumtemperatur blockiert.

Das Decapeptid mit der in SEQ ID NO 3 gezeigten Sequenz, das gemäß Beispiel 1b) hergestellt wurde, wird aminoterminal mit D-Biotinyl-εamidocapronsäure-N-succinimidester (Boehringer Mannheim, Katalog Nr. 1008960) gemäß Angaben des Herstellers biotinyliert. Das biotinylierte Peptid wird in PBS, 0,05 % Tween 20, 1 % BSA in einer Konzentration von 10 ng/ml gelöst und durch 1-stündige Inkubation von 100 µl je Vertiefung an die mit Streptavidin beschichtete Mikrotiterplatte gebunden. Anschließend wird ungebundenes Peptid durch dreimaliges Waschen mit PBS, 0,05 % Tween 20 entfernt.

Jeweils 150 µl der zu untersuchenden Probe (Serum, Plasma oder ein Standard) werden mit 150 µl des Antikörpers nach Beispiel 4 für 2 Stunden bei 37°C (oder über Nacht bei 4°C) inkubiert. Jeweils 100 µl dieses Ansatzes werden in die Vertiefungen der Mikrotiterplatte zu dem gebundenen Decapeptid gegeben und 60 Minuten bei 37 °C inkubiert. Dabei kann nur der nach der Inkubation mit der Probe noch nicht gebundene Antikörperüberschuß des Antiserums an das immobilisierte Decapeptid binden.

Nach dreimaligem Waschen mit PBS/0,05 % Tween 20 wird gebundener Antikörper durch anschließende Inkubation mit einem Anti-Kaninchen-IgG-POD Konjugat (Boehringer Mannheim GmbH, Katalog Nr. 1238 850) und ABTS® (1 mg/ml) nachgewiesen.

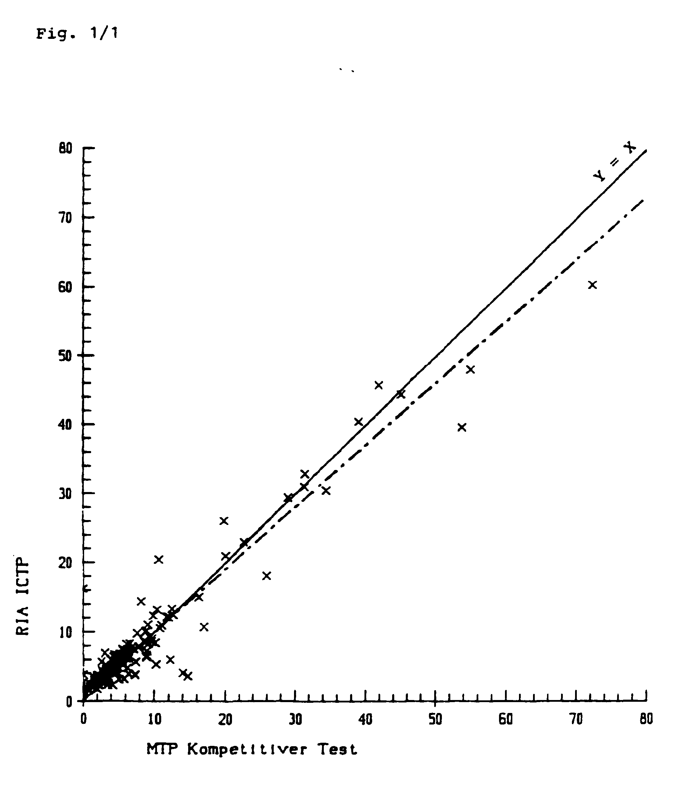

Mit dem Test (MTP Kompetitiver Test) wurden 164 Patienten-Seren vermessen. Die Ergebnisse wurden zu Daten, welche mit einem Radioimmunoassay (RIA) ermittelt wurden, in Bezug gesetzt. Dieser RIA ICTP (Telopeptide ICTP [¹²⁵J] von Orion Diagnostica, Finnland) basiert auf quervernetzten Kollagenfragmenten, welche durch enzymatischen Verdau und biochemische Verfahren hergestellt und isoliert werden. Aus Figur 1 geht nun hervor, daß das Verfahren Meßwerte erbringt, die gut mit den RIA-Werten korrelieren, d.h. das Verfahren liefert klinisch relevante Daten. Es wurde ein Korrelationskoeffizient von 0,959 ermittelt.

## Patentansprüche

1. Kompetitiver Immunoassay zum Nachweis von Kollagen Typ I oder Kollagenfragmenten davon in einer Probe, **dadurch gekennzeichnet, dass** ein Bindepartner, der ein synthetisches lineares Peptid enthält, das einer Sequenz des nicht-helikalen N-terminalen Bereichs von Kollagen Typ I oder der Sequenz SEQ ID NO 1 aus dem nicht-helikalen C-terminalen Bereich von Kollagen Typ I entspricht und keine Hydroxylysyl- oder Lysylpyridinolin-Quervernetzung enthält,
mit einem Antikörper, der das synthetische lineare Peptid zu binden vermag,
und der Probe inkubiert,
und die Bindung des Antikörpers an den Bindepartner in geeigneter Weise bestimmt wird.

2. Verwendung eines Antigens, das ein synthetisches lineares Peptid enthält, das einer Sequenz des nicht-helikalen N-terminalen Bereichs von Kollagen Typ I oder der Sequenz SEQ ID NO 1 aus dem nicht-helikalen C-terminalen Bereich von Kollagen Typ I entspricht und keine Hydroxylysyl- oder Lysylpyridinolin-Quervernetzung enthält, als Standardmaterial zur Erstellung einer Standard- oder Eichkurve in einem kompetitiven Immunoassay zum Nachweis von Kollagen Typ I oder Kollagenfragmenten davon.

## Claims

1. Competitive immunoassay for the detection of collagen type I or collagen fragments thereof in a sample, **characterized in that** a binding partner which contains a synthetic linear peptide which corresponds to a sequence of the non-helical N-terminal region of collagen type I or to the sequence SEQ ID NO 1 from the non-helical C-terminal region of collagen type I and contains no hydroxylysyl or lysylpyridinoline cross-linking,
is incubated with an antibody that is able to bind the synthetic linear peptide and with the sample,
and the binding of the antibody to the binding partner is determined in a suitable manner.

2. Use of an antigen which contains a synthetic linear peptide which corresponds to a sequence of the non-helical N-terminal region of collagen type I or to the sequence SEQ ID NO 1 from the non-helical C-terminal region of collagen type I and contains no hydroxylysyl or lysylpyridinoline cross-linking, as a standard material for preparing a standard curve or calibration curve in a competitive immunoassay for the detection of collagen type I or collagen fragments thereof.

## Revendications

1. Dosage immunologique par compétition pour détecter le collagène de type 1 ou des fragments de collagène de ce type dans un échantillon, **caractérisé en ce qu'**un partenaire de liaison, qui contient un peptide linéaire synthétique, lequel correspond à une séquence de la région N-terminale non hélicoïdale du collagène de type 1 ou à la séquence SEQ ID N° 1 de la région C-terminale non hélicoïdale du collagène de type 1 et ne contient aucune réticulation transversale hydroxylysyle ou lysylpyridinoline,
est incubé avec un anticorps, qui est capable de se lier au peptide linéaire synthétique,
et avec l'échantillon,
et la liaison de l'anticorps au partenaire de liaison est déterminée de manière appropriée.

2. Utilisation d'un antigène, qui contient un peptide linéaire synthétique, lequel correspond à une séquence de la région N-terminale non hélicoïdale du collagène de type 1 ou à la séquence SEQ ID N° 1 de la région C-terminale non hélicoïdale du collagène de type 1 et ne contient aucune réticulation transversale hydroxylysyle ou lysylpyridinoline, en tant que matière étalon pour le traçage d'une courbe des moyennes ou d'une courbe d'étalonnage dans un dosage immunologique par compétition pour détecter le collagène de type 1 ou des fragments de collagène de ce type.
